# EUROPÄISCHE PATENTANMELDUNG

(11) **EP 0 542 126 A2**
(43) Veröffentlichungstag der Anmeldung: **19.05.1993**
(21) Anmeldenummer: 92118930.4
(22) Anmeldetag: 05.11.1992
(51) Int. Cl.: A61B 17/04

(54) **Chirurgische Nähvorrichtung**

(30) Priorität: 13.11.1991 DE 4137218
(71) Anmelder: Heidmüller, Harald, D-51061 Köln (DE)
(72) Erfinder: Heidmüller, Elke, W-5000 Köln 80 (DE)
(74) Vertreter: Selting, Günther, Dipl.-Ing.

(57) **Zusammenfassung**

Zum Vernähen einer Operationsöffnung (32) ist ein Instrument (10) vorgesehen, das in die Operationsöffnung eingeführt werden kann. Aus dem Instrument (10) werden Nadelhalter mit daran angebrachten Nadeln (25) seitlich ausgefahren. Durch Anheben des Instruments (10) werden die Nadeln (25) von innen nach außen durch das Hautgewebe (33) hindurchgestochen, wobei der durch die Nadeln (25) hindurchgehende Faden (30) im Innern des Patientenkörpers eine Schlinge bildet. Mit einem Nadelfänger (26) werden die Nadeln aus dem Patientenkörper herausgezogen, wobei sie sich von den Nadelhaltern (19) lösen. Die Fadenschleife kann schließlich über der Operationsöffnung außerhalb des Patientenkörpers zugeknotet werden.

## Beschreibung

Die Erfindung betrifft eine chirurgische Nähvorrichtung zum Schließen einer Operationswunde, bzw. einer Stichincision mit mindestens einem Faden.

Eine chirurgische Nähvorrichtung nach dem Oberbegriff des Patentanspruchs 1 ist bekannt aus US 4 493 323. Die bekannte Vorrichtung weist ein Instrument mit einem langgestreckten Gehäuse von ovalem Querschnitt auf, in dem zwei parallele Nadelhalter in Längsrichtung verschiebbar sind. In jeden der Nadelhalter kann eine Nadel eingesetzt werden, derart, daß die beiden Nadeln zum patientenseitigen Ende vorstehen. Durch die rückwärtigen Enden der Nadeln wird der Operationsfaden hindurchgezogen, so daß er eine weiträumige Schleife bildet. Beim Gebrauch des Instruments wird dieses mit einem Stechwerkzeug in den Körper bzw. in das Knie eines Patienten eingeführt und die Nadeln werden vorgeschoben, wobei sie den zu nähenden Bereich seitlich umschließen. Dann werden die Nadeln weitergeschoben, bis sie auf der gegenüberliegenden Seite wieder aus dem Körper des Patienten austreten. Dort können die Fadenenden ergriffen und über der Haut verknotet werden. Diese Nähvorrichtung setzt voraus, daß die Nadeln vollständig durch den Körper hindurchgestochen werden, d.h. an dem dem Einstichende gegenüberliegenden Ende wieder austreten. Das Zunähen von Stichincisionen, die nur von einer Seite aus zugänglich sind, ist mit dem bekannten Gerät nicht möglich.

Bei chirurgischen Operationen, z.B. im Bauch- und Thoraxbereich, wird die Operationsnaht durch Nähen mit chirurgischem Nahtmaterial verschlossen. Bei relativ langen Operationsschnitten bereitet die Herstellung der Operationsnaht in der Regel keine größeren Probleme. Schwierigkeiten ergeben sich dagegen bei Stichincisionen, insbesondere im Bereich der Bauchhöhle und des Thorax. Wenn der Operationsschnitt nur so kurz ist, daß die Naht nur mit mindestens einer Fadenschlinge genäht werden kann, bereitet es dem Operateur Schwierigkeiten, die Nadel mit Nadelhalter unter die Haut zu führen und sie von unten her durch die Bauchdecke hindurchzustecken, weil er mit den Fingern nicht durch die Schnittöffnung hindurchreichen kann.

Der Erfindung liegt die Aufgabe zugrunde, eine chirurgische Nähvorrichtung zu schaffen, die das Zunähen von Stichincisionen ermöglicht, ohne daß der Operateur durch den Schnittbereich hindurch mit den Fingern oder mit bekannten chirurgischen Instrumenten in den Körper des Patienten eingreifen muß.

Die Lösung dieser Aufgabe erfolgt erfindungsgemäß mit den im Patentanspruch 1 angegebenen Merkmalen.

Die erfindungsgemäße Nähvorrichtung weist ein Instrument auf, an dessen unterem Ende mindestens zwei Nadelhalter vorgesehen sind, an denen chirurgische Nadeln mit nach oben weisenden Spitzen angebracht werden können. Das Instrument wird in die Operationswunde eingeführt und anschließend herausgezogen, während die Nadeln in seitlichem Abstand von ihm angeordnet sind. Dabei durchdringen die nach oben weisenden Nadelspitzen gleichzeitig die Ränder der zu vernähenden Gewebeschichten. Ein durch Löcher der Nadeln hindurchgeführter Nahtmaterialfaden bildet im Innern des Patientenkörpers eine Schleife oder Schlinge, während beide Fadenenden durch die Nadeln aus dem Patientenkörper herausbewegt werden. Die Fadenenden können dann ergriffen werden und der Faden kann zum Schließen der Operationswunde von außen festgezogen und verknotet werden.

Die erfindungsgemäße Nähvorrichtung erlaubt das Zusammennähen zweier Wundränder durch Erzeugung einer geschlossenen Fadenschleife in einem einzigen Arbeitsgang, ohne daß der Operateur mit den Fingern in den Körper des Patienten eingreifen muß. Wenn die Nadeln das zu vernähende Gewebe des Patienten von innen nach außen durchstochen haben, werden sie ergriffen und von den Nadelhaltern, an denen sie klemmend festgehalten werden, abgezogen. Das Instrument wird so gehandhabt, daß die Nadeln, nachdem sie gegebenenfalls das Hautgewebe von innen nach außen durchstochen haben, außen abgezogen und über die freien Fadenenden hinweg abgestreift werden. Anstelle der Verwendung eines Nadelfängers können die Nadeln auch mit der Hand oder einem anderen Instrument erfaßt werden.

Ein besonderer Vorteil besteht in der einfachen Handhabbarkeit der Nähvorrichtung, die kein spezielles Geschick und keine große Erfahrung beim Vernähen von Operationswunden voraussetzt. Das Instrument wird lediglich in die Operationswunde eingeführt. Erforderlichenfalls werden dann die Nadelhalter z.B. seitlich ausgestellt, so daß die Nadeln einen Abstand von der Stabwand erhalten. Danach wird der Stab ein Stück zurückgezogen, wobei die Nadeln das Gewebe in der Nähe der Wundränder durchdringen. Schließlich können die Nadeln von dem Nadelfänger oder mit der Hand oder einem anderen Instrument erfaßt und aus dem Patientenkörper herausgezogen werden. Danach erfolgt das Herausziehen der Nähvorrichtung aus dem Patientenkörper und schließlich das Verknoten der Fadenschlinge außerhalb des Patientenkörpers.

Bei einer bevorzugten Ausführungsform der Nähvorrichtung sind die Nadelhalter quer zum Instrument bewegbar geführt, wobei längs des stabförmigen Instrumentes ein Betätigungsmechanismus zur seitlichen Bewegung der Nadelhalter verläuft. Diese Ausführungsform bietet den Vorteil, daß die Nadeln im Einführzustand des schmalen Instruments eng an diesem anliegen oder in die Außenkontur des Instruments versenkt sind und nicht vorstehen. Erst wenn die Nadeln sich unterhalb des zu vernähenden Gewebes befinden, werden die Nadelhalter durch den Betätigungsmechanismus seitlich ausgefahren. Es ist allerdings auch möglich, die Nadelhalter in einem gewissen Abstand vom Instrument fest anzubringen, wobei das Instrument nach dem Eindringen in die Operationswunde um etwa 90° gedreht werden muß, damit die Nadeln in den Bereich der Seitenränder der Operationswunde gelangen.

Die Merkmale des Anspruches 6 sind vorteilhaft. Der kegelstumpfförmige Nadelfänger ermöglicht, daß gegebenenfalls Haut und Fettgewebe durch seine konische Form verdrängt und somit nur das Nähen der Fascie und des Peritoneums ermöglicht wird.

Im folgenden werden unter Bezugnahme auf die Zeichnungen Ausführungsbeispiele der Erfindung näher erläutert.

Es zeigen:
- Fig. 1: eine Seitenansicht der Nähvorrichtung, teilweise geschnitten, im unbetätigten Zustand während des Einführens des Stabes in den Körper des Patienten,
- Fig. 2: das seitliche Fortbewegen der Nadelhalter von dem Stab durch den Betätigungsmechanismus,
- Fig. 3: die Perforation des Gewebes von innen her,
- Fig. 4: das Einsetzen des Nadelfängers zum Festhalten der Nadelspitzen von außen,
- Fig. 5: das Herausziehen des Gerätes aus dem Patientenkörper,
- Fig. 6: eine zweite Ausführungsform der Vorrichtung im zusammengelegten Einführzustand und
- Fig. 7: die Vorrichtung nach Fig. 6 im auseinandergeklappten Zustand.

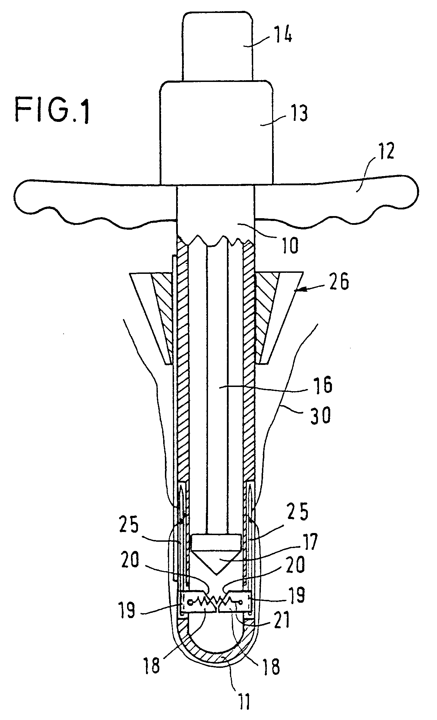

Das in den Fign. 1-5 dargestellte Gerät weist ein langgestrecktes Instrument in Form eines geraden Stabes auf, der hier als hohles Rohr ausgebildet ist, dessen vorderes Ende 11 mit einer abgerundeten Kuppe verschlossen ist. Am rückwärtigen Ende des Instruments 10 befindet sich ein Handgriff 12, mit dem das Instrument 10 geführt werden kann. Am Handgriff ist ein Führungsstück 13 angebracht, in dem ein Betätigungsknopf 14 verschiebbar ist und das eine Feder 15 enthält, welche den Betätigungsknopf 14 nach außen vorspannt. Der Betätigungsknopf 14 ist mit einer Stange 16 verbunden, die in dem hohlen Stab verläuft und die am unteren Ende einen Betätigungskopf 17 trägt.

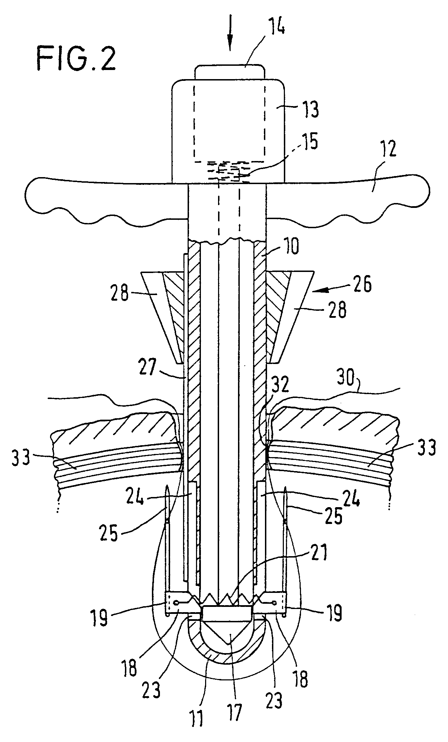

Am unteren Ende des Stabes sind zwei Gleitstücke 18 vorgesehen, die quer zur Längsrichtung des Stabes geführt sind. Jedes dieser Gleitstücke 18 weist einen Nadelhalter 19 auf. Die Gleitstücke 18 haben an den einander zugewandten Enden Schrägflächen 20, die einen V-förmigen Spalt im Innern des Instruments 10 bilden. In diesen Spalt kann der keilförmige Betätigungskopf 17 eindringen, um die Gleitstücke 18 seitlich auseinanderzubewegen. Die Gleitstücke 18 sind durch eine Federvorrichtung 21 in der Weise gespannt, daß sie in das Instrument 10 eingezogen werden. Wenn der Betätigungsknopf 14 gedrückt wird und dadurch der Betätigungskopf 17 die Gleitstücke 18 auseinandertreibt, werden die Nadelhalter 19 seitlich aus dem Instrument 10 herausbewegt, so wie dies in Fig. 2 dargestellt ist. Die Steuerung der beiden Nadelhalter 19 durch den Betätigungskopf 17 erfolgt synchron, d.h. beide Nadelhalter werden symmetrisch zueinander in Bezug auf die Stabachse bewegt.

Die Gleitstücke 18 ragen durch Löcher 23 der Stabwand hindurch. Oberhalb jedes Lochs 23 befindet sich eine längslaufende Nut 24 an der Außenseite des Instruments 10. Die Nuten 24 dienen zur Aufnahme der Nadeln 25 in dem in Fig. 1 dargestellten Zustand.

Längs des Instruments 10 ist der Nadelfänger 26 verschiebbar. Eine am Stab 10 längslaufende Leiste 27, die in eine entsprechende Nut des Nadelfängers 26 eindringt, verhindert Verdrehungen des Nadelfängers 26 in Bezug auf das Instrument 10. Der Nadelfänger 26 ist kegelstumpfförmig ausgebildet, wobei er sich nach oben (von dem Ende 11 fort) erweitert. An zwei entgegengesetzten Seiten sind Klemmschlitze 28 am Nadelfänger 26 vorgesehen, deren Grund entsprechend der Orientierung und dem Winkel der Konizität des Nadelfängers 26 konisch verlaufen kann und in denen die Nadeln 25 festgeklemmt werden können. Anstelle der Klemmschlitze 28 können auch andere Klemmvorrichtungen, in die die Nadelspitzen eindringen können, verwendet werden, insbesondere solche, die ein ungehindertes Eindringen der Nadeln ermöglichen, das Herausziehen aber nur nach Betätigen eines Handgriffs zulassen.

Die Nadeln 25 sind mit ihren stumpfen unteren Enden in die Nadelhalter 19 eingesetzt, während ihre Spitzen nach oben zeigen. Etwa im mittleren Bereich der Nadellänge ist jeweils ein Nadelöhr vorgesehen, durch das der Faden 30 hindurchgezogen werden kann. Die Nadeln können auch schräg zum Instrument 10 ausgerichtet sein.

Bei Benutzung der Nähvorrichtung verläuft der Faden 30 durch die Nadelöhre beider Nadeln 25 hindurch, so daß die beiden Fadenenden nach oben frei abstehen. Die Nadeln 25 sind seitlich in das Instrument 10 eingezogen oder an diesen herangezogen. In diesem Zustand, der in Fig. 1 dargestellt ist, wird die Nähvorrichtung durch eine Stichincision in den Patientenkörper eingeführt.

In Fig. 2 ist die Schnittöffnung der Stichincision mit 32 bezeichnet und das angrenzende Gewebe ist mit 33 bezeichnet. Dieses Gewebe 33 besteht in bekannter Weise aus mehreren Schichten, nämlich dem Peritoneum, dem Fasziengewebe und dem darüberliegenden Fett- und Hautgewebe.

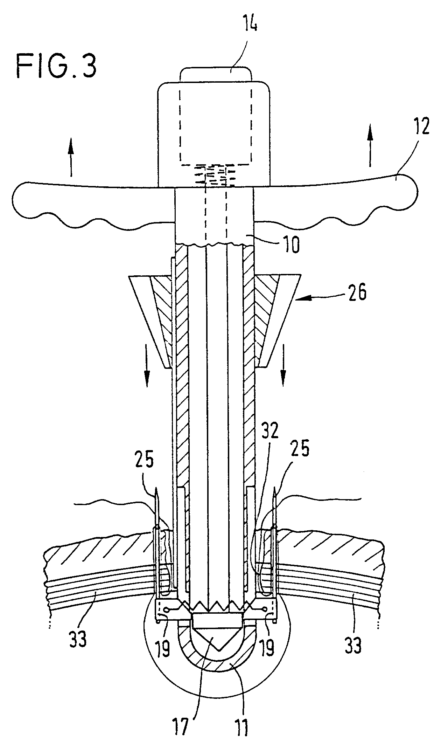

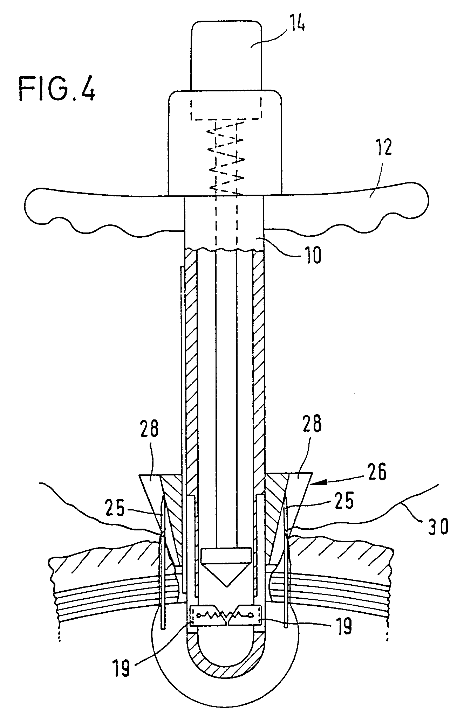

Durch Niederdrücken des Betätigungsknopfes 14 werden die Nadelhalter 19 gemäß Fig. 2 seitlich aus dem Instrument 10 herausbewegt. Durch einen (nicht dargestellten) Rastmechanismus kann der Betätigungsknopf in dieser Stellung festgehalten werden, so daß es nicht erforderlich ist, ihn weiterhin manuell gedrückt zu halten. Dann wird das Instrument 13 an dem Handgriff 12 gemäß Fig. 3 hochgezogen, wobei die Nadeln 25 mit ihren Spitzen den Rand der Körperöffnung 32 von innen nach außen durchstoßen, so daß die Nadelspitzen aus dem zu nähenden Gewebe herausragen. Nun wird gemäß Fig. 4 der Nadelfänger 26 vom Operateur längs des Instruments 10 nach unten geschoben, bis die Spitzen der Nadeln in die Klemmschlitze 28 eindringen und von diesen festgehalten werden. In diesem Zustand kann der Nadelfänger 26 unter Mitnahme der Nadeln 25 hochgezogen werden, wobei sich die Nadeln von den Nadelhaltern 19 lösen. Dies liegt daran, daß der Nadelfänger 26 die Nadeln fester halten kann als die Nadelhalter 19 dies vermögen. Es ist allerdings auch möglich, gemäß Fig. 4 zunächst die Nadelhalter 19 einzuziehen, wobei sie sich von den Nadeln 25 lösen, und erst dann den Nadelfänger 26 entlang des Instruments 10 hochzuschieben. Durch die konische Form des Nadelfängers 26 werden die Haut und das Fettgewebe beiseitegeschoben und die Nadelspitzen werden oberhalb des Fasziengewebes vom Nadelfänger erfaßt.

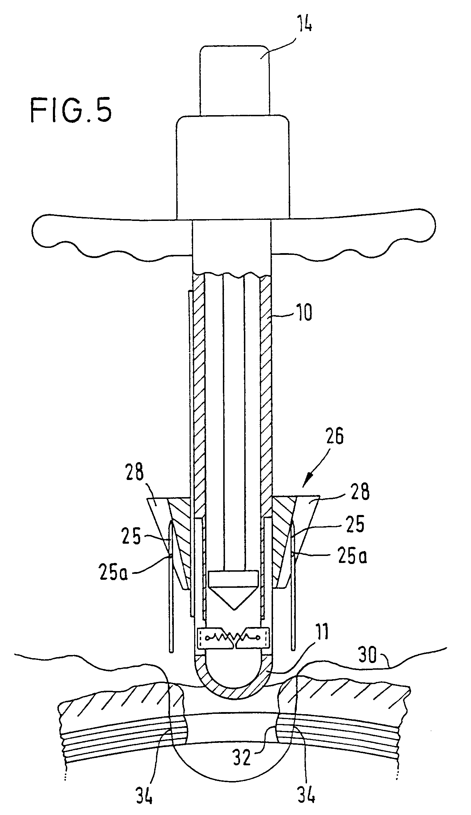

Fig. 5 zeigt den Zustand, daß die Nadeln 25 am Nadelfänger 26 festsitzen und zusammen mit diesem und dem Instrument 10 aus der Stichincision 32 herausgezogen werden. Dabei gleitet jeweils ein Abschnitt des Fadens 30 durch das betreffende Nadelöhr 25a der zugehörigen Nadel 25, so daß die Nadeln schließlich von dem Faden 30 freikommen. Der Faden 30, der nun durch die beiden Stichkanäle 34 beidseitig der Operationsöffnung 32 hindurchführt und unter der Operationsöffnung eine Schleife bildet, kann nun über der Operationsöffnung 32 verknotet werden, wobei die Operationsöffnung zusammengezogen wird.

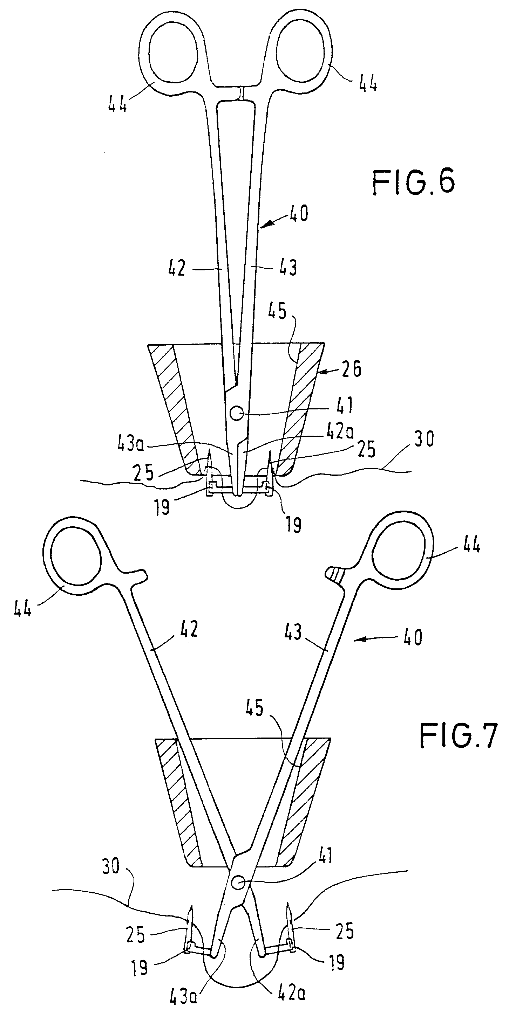

Bei dem Ausführungsbeispiel der Fign. 6 und 7 besteht das Instrument 40 aus einer Zange mit zwei sich in einem Gelenk 41 kreuzenden Zangenbeinen 42,43, die an ihren langen Griffschenkeln Griffteile 44 und an ihren kurzen Arbeitsschenkeln 42a bzw. 43a nach außen, d.h. entgegengesetzt, gerichtete und von den Arbeitsschenkeln rechtwinklig abstehende Nadelhalter 19 aufweisen. In diese Nadelhalter 19 sind die Nadeln 25 mit zu den Griffteilen 44 gerichteten Spitzen klemmend eingesetzt. Die Zangenschenkel 42,43 führen durch einen Nadelfänger 26 hindurch, der einen entsprechenden schlitzförmigen Längskanal 45 aufweist und längs des Instruments 40 verschiebbar ist.

Das Instrument 40 wird in dem in Fig. 6 dargestellten Zustand in die Operationsöffnung des Körpers eingeführt. Dann werden die Zangenschenkel 42,43 auseinanderbewegt, so daß der gegenseitige Abstand der Nadeln 25 sich vergrößert. In diesem Zustand, der in Fig. 7 dargestellt ist, wird das Instrument hochgezogen, wobei die Nadeln 25 das Körpergewebe durchdringen. Die aus der Haut herausstehenden Nadelspitzen werden dann durch Vorschieben des Nadelfängers 26 von diesem ergriffen und die Nadeln 25 werden von den Nadelhaltern abgezogen. Schließlich wird das Instrument 40 mit gegeneinandergelegten Griffschenkeln und Arbeitsschenkeln aus der Operationswunde herausgezogen und danach wird der Nahtmaterialfaden 30 außerhalb des Patientenkörpers verknotet, um die Operationswunde zu schließen.

## Patentansprüche

1. Chirurgische Nähvorrichtung, bestehend aus einem Instrument (10), das am patientenseitigen Ende (11) mindestens zwei Nadelhalter (19) trägt, in die jeweils eine Nadel (25) mit von diesem Ende (11) fortweisender Spitze einsetzbar ist,
**dadurch gekennzeichnet,**
daß die Nadelhalter (19) nach entgegengesetzten Seiten seitlich von dem Instrument (10) abstehen oder aus diesem herausbewegbar sind und daß die Spitzen der Nadeln (25) zum patientenfernen Ende des Instruments gerichtet sind.

2. Chirurgische Nähvorrichtung nach Anspruch 1, gekennzeichnet durch einen entlang des Instruments (10) verschiebbaren Nadelfänger (26) zum Festhalten der Nadeln (25) und zum Abziehen der Nadeln von den Nadelhaltern (19).

3. Chirurgische Nähvorrichtung nach Anspruch 1 oder 2, dadurch gekennzeichnet, daß die Nadelhalter (19) an dem stabförmigen Instrument (10) in Querrichtung bewegbar geführt sind, und daß längs des Instruments (10) ein Betätigungsmechanismus (14,16,17) zum seitlichen Bewegen der Nadelhalter (19) verläuft.

4. Chirurgische Nähvorrichtung nach Anspruch 3, dadurch gekennzeichnet, daß der Betätigungsmechanismus eine Federvorrichtung (21) zum Heranziehen der Nadelhalter (19) an das Instrument (10) aufweist.

5. Chirurgische Nähvorrichtung nach einem der Ansprüche 1-4, dadurch gekennzeichnet, daß das Instrument (10) als hohles Rohr ausgebildet ist, in dem eine längsbewegbare Stange (16) des Betätigungsmechanismus verläuft.

6. Chirurgische Nähvorrichtung nach einem der Ansprüche 1-5, dadurch gekennzeichnet, daß der Nadelfänger (26) kegelstumpfförmig gestaltet und auf dem Instrument (10) unverdrehbar geführt ist und für jede Nadel (25) eine Klemmöffnung (28) aufweist.

7. Chirurgische Nähvorrichtung nach einem der vorhergehenden Ansprüche, dadurch gekennzeichnet, daß das Instrument (40) auseinanderschwenkbare Zangenschenkel (42a,43a) aufweist, an denen die Nadelhalter (19) angebracht sind.
